# EUROPÄISCHE PATENTSCHRIFT

(11) **EP 4 311 505 B1**
(45) Veröffentlichungstag und Bekanntmachung des Hinweises auf die Patenterteilung: **11.11.2026**
(21) Anmeldenummer: 23184047.1
(22) Anmeldetag: 07.07.2023
(51) Int. Cl.: A61B 17/15

(54) **CHIRURGISCHES INSTRUMENT**
SURGICAL INSTRUMENT
INSTRUMENT CHIRURGICAL

(30) Priorität: 25.07.2022 DE 102022207575
(43) Veröffentlichungstag der Anmeldung: 31.01.2024
(73) Patentinhaber: Aesculap AG, 78532 Tuttlingen (DE)
(72) Erfinder: Firmbach, Franz-Peter, 78576 Emmingen-Liptingen (DE); Stoffels, Lilli, 75365 Calw (DE)
(74) Vertreter: Patentanwälte Ruff, Wilhelm, Beier, Dauster & Partner mbB

(56) Entgegenhaltungen:
- AU-A1- 2010 283 577
- US-A1- 2015 045 801
- US-A1- 2021 000 484
- US-B2- 10 166 034

## Beschreibung

Die Erfindung betrifft ein chirurgisches Instrument zur Verwendung bei einer Kniegelenkersatzoperation.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs oder/und der Tibia durch künstliche Gelenkflächen einer Kniegelenkprothese ersetzt. Solche Kniegelenkprothesen umfassen üblicherweise eine Femurkomponente und eine Tibiakomponente. Die Femurkomponente wird am distalen Ende des Femurs implantiert. Die Tibiakomponente wird am proximalen Ende der Tibia implantiert.

Vor der Implantation der prothetischen Komponenten werden der distale Femur und die proximale Tibia reseziert. Hierfür bringt der Chirurg unterschiedliche Resektionsschnitte an und trennt Knochen- oder/und Knorpelmaterial von dem jeweiligen Knochen ab. Durch die Resektion wird der jeweilige Knochen in seiner Form an die aufzunehmende prothetische Komponente angepasst.

Die Resektion kann auf der Grundlage unterschiedlicher Konzepte ausgeführt werden. Ein Konzept zielt darauf ab, die Spannungen der Bänder des Knies bei der Gelenkbewegung ausgeglichen zu halten. Hierdurch soll eine bessere Funktion der Kniegelenkprothese gewährleistet werden. Dieses Konzept wird allgemein als "gap balancing" bezeichnet. Bei anderen Konzepten entfernt der Chirurg mittels der Resektion eine bestimmte Menge an Knochen- oder/und Knorpelmaterial. Solche Konzepte werden allgemein als "measured resection" bezeichnet. Die Ausrichtung der Resektionsschnitte in Bezug auf die Anatomie des Patienten bestimmt die spätere Ausrichtung der implantierten Komponenten und folglich auch die Orientierung der prothetischen Gelenkachsen. Die Ausrichtung der Resektionsschnitte ist daher von besonderer Bedeutung.

Bei der Ausrichtung der Resektionsschnitte werden in erster Linie drei Ansätze unterschieden: mechanisch, anatomisch und kinematisch. Bei der mechanischen Ausrichtung wird die proximale Tibia senkrecht zur Längsachse des Tibiaschafts reseziert. Die Resektion des distalen Femurs erfolgt entsprechend hieran angepasst. Erforderlichenfalls werden Bandentlastungen (englisch: ligament releases) durchgeführt. Bei der anatomischen Ausrichtung wird versucht, die Tibia in einem Varus-Winkel von 3° zu resezieren. Die Femurresektion und Bandentlastungen werden mit dem Ziel einer geraden Hüft-Knie-Knöchel-Achse des Beins durchgeführt. Ziel der kinematischen Ausrichtung (englisch: kinematic alignment, nachfolgend abgekürzt als KA) ist es, die künstlichen Gelenkflächen der prothetischen Komponenten auf dem Niveau der präarthritischen, defektfreien natürlichen Gelenkflächen zu implantieren.

Bei einem KA erfolgt die Ausrichtung der Resektionsschnitte oftmals ausgehend von dem distalen Femur. Die Resektion der proximalen Tibia erfolgt hieran angepasst. Zum Zweck der zeitlich nacheinander erfolgenden Resektion von Femur und Tibia sind spezielle chirurgische Instrumente bekannt, die auch als tibiales Ausricht- oder/und Übertragungswerkzeug (tibial cut alignment guide) bezeichnet werden. Solche Instrumente erlauben eine Übertragung der Ausrichtung der femoralen Resektionsschnitte auf die Tibia. Die Übertragung erfolgt üblicherweise nach einer wenigstens distalen Resektion des Femurs, bei welcher die distalen Kondylen abgetrennt werden. Die Übertragung kann in Extension oder Flexion erfolgen. Bei einer Variante der KA wird zunächst der distale Femur vollständig präpariert (all femur first). Dabei werden die am distalen Femur vorzunehmenden Resektionsschnitte an der Anatomie des Patienten ausgerichtet und am distalen Femur angebracht. Anschließend wird ein femorales Probekondylenimplantat an dem distalen Femur angebracht. Das bereits nach der Anatomie des Patienten ausgerichtete femorale Probekondylenimplantat wird dann als Referenzkomponente herangezogen, deren Ausrichtung mittels des chirurgischen Instruments oder Übertragungswerkzeugs auf die proximale Tibia übertragen wird, um die daran vorzunehmenden Resektionsschnitte auszurichten und anzubringen. Es versteht sich, dass alternativ ein Vorgehen in umgekehrter Reihenfolge denkbar ist, also von der proximalen Tibia ausgehend.

Chirurgische Instrumente für ein KA weisen üblicherweise eine erste Befestigungseinrichtung auf, die zur lösbaren Befestigung an einer femoralen oder tibialen Referenzkomponente eingerichtet ist. Typischerweise ist eine Führungseinrichtung vorhanden, an welcher ein tibialer oder femoraler Schnittblock befestigbar ist. Das heißt, dass ein solches chirurgische Instrument prinzipiell entweder zusammen mit einer femoralen Referenzkomponente und einem tibialen Schnittblock oder alternativ zusammen mit einer tibialen Referenzkomponente und einem femoralen Schnittblock verwendet werden kann. Die Führungseinrichtung erlaubt eine geführte Relativbewegung zwischen der Referenzkomponente und dem Schnittblock.

Vor einer erneuten Verwendung muss das chirurgische Instrument üblicherweise gereinigt, insbesondere sterilisiert, werden. Vor allem im Bereich der Führungseinrichtung sind oftmals schwer zugängliche Stellen vorhanden, die ohne ein Trennen der Führungseinrichtung von den übrigen Komponenten des chirurgischen Instruments für einen Reinigungsvorgang nur erschwert oder gar nicht erreichbar sind. Wünschenswerterweise lässt sich das chirurgische

Instrument vor diesem Hintergrund wenigstens teilweise in seine Komponenten zerlegen. Dokument US2015/045801 A1 offenbart ein femoral referenziertes tibiales Ausrichtungssystem für eine Kniearthroplastie, insbesondere mit einem femoralen Referenzelement, einem Resektionsführungsturm, einem tibialen Resektionsführungselement und einem Verlängerungsteil.

Aufgabe der Erfindung ist es, ein chirurgisches Instrument bereitzustellen, welches Vorteile gegenüber herkömmlichen chirurgischen Instrumenten bietet und insbesondere ohne gesondertes Montagewerkzeug wenigstens teilweise in seine Komponenten zerlegbar ist.

Diese Aufgabe wird durch das Bereitstellen eines chirurgischen Instruments mit den Merkmalen des Anspruchs 1 gelöst. Bevorzugte Ausführungsformen sind Gegenstand der abhängigen Patentansprüche.

Das erfindungsgemäße chirurgische Instrument dient zur Verwendung bei einer Kniegelenkersatzoperation und weist auf: eine erste Befestigungseinrichtung, welche zur lösbaren Befestigung an einer femoralen Referenzkomponente eingerichtet ist, eine Führungseinrichtung, welche zum einen mit der ersten Befestigungseinrichtung lösbar gekoppelt und zum anderen mit einer zweiten Befestigungseinrichtung, welche zur lösbaren Befestigung an einer tibialen Komponente eingerichtet ist, koppelbar ist, und eine in einer sagittalen Führungsebene geführte Relativbewegung zwischen der ersten Befestigungseinrichtung und der zweiten Befestigungseinrichtung ermöglicht, wobei die Führungseinrichtung eine Kulissenführung mit wenigstens einem entlang einer Führungsbahn längserstreckten Kulissenschlitz und wenigstens einem Kulissenelement aufweist, welches entlang der Führungsbahn gleitbeweglich in dem Kulissenschlitz aufgenommen ist, und wobei die erste Befestigungseinrichtung wenigstens eine orthogonal zur Führungsbahn erstreckte Steckaufnahme aufweist, durch welche das wenigstens eine Kulissenelement unter Ausbildung einer lösbaren Steckverbindung mit der ersten Befestigungseinrichtung in den wenigstens einen Kulissenschlitz eingebracht ist. Die erste Befestigungseinrichtung kann - alternativ oder zusätzlich - zur lösbaren Befestigung an einer tibialen Referenzkomponente eingerichtet sind. Die zweite Befestigungseinrichtung kann - alternativ oder zusätzlich - zur lösbaren Befestigung an einer femoralen Komponente eingerichtet sein. Das chirurgische Instrument ist also bei einem von der Tibia ausgehenden KA oder/und bei einem von dem Femur ausgehenden KA verwendbar. Weder die femorale Referenzkomponente noch die tibiale Komponente noch die zweite Befestigungseinrichtung sind Bestandteil des chirurgischen Instruments. Die Führungsbahn ist vorzugsweise in der oder parallel zur Führungsebene angeordnet. Das wenigstens eine Kulissenelement dient zur Kopplung der ersten Befestigungseinrichtung mit der Führungseinrichtung. Vorteilhaft erfüllt das wenigstens eine Kulissenelement somit zwei Funktionen gleichermaßen. Denn es ermöglicht einerseits die gleitbewegliche Verbindung der ersten Befestigungseinrichtung mit der Führungseinrichtung. Andererseits erlaubt es zudem eine besonders einfache Zerlegung des chirurgischen Instruments in seine Komponenten, indem es unter Lösung der Steckverbindung aus dem Kulissenschlitz genommen wird. Zweckmäßig lässt sich die Steckverbindung manuell, insbesondere werkzeugfrei, lösen oder/und zusammenfügen. Somit lässt sich das chirurgische Instrument besonders einfach und gründlich reinigen. Außerdem lässt sich das chirurgische Instrument erforderlichenfalls während der Kniegelenkersatzoperation einfach manuell zerlegen oder zusammenbauen,. Entsprechend kann die erste Befestigungseinrichtung an der bereits am jeweiligen Knochen montierten Referenzkomponente gesondert befestigt werden, bevor die Führungseinrichtung mittels der Steckverbindung an der ersten Befestigungseinrichtung montiert wird. Hieraus ergeben sich Vorteile in der Handhabung des chirurgischen Instruments.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet.

In Ausgestaltung der Erfindung weist das wenigstens eine Kulissenelement einen Gleit- und einen Steckabschnitt auf, wobei zur gleitbeweglichen Führung entlang der Führungsbahn der Gleitabschnitt in den Kulissenschlitz eingreift und der Steckabschnitt in einer zugehörigen Aufnahmeöffnung der Steckaufnahme lösbar aufgenommen ist. Bevorzugt ist das wenigstens eine Kulissenelement zwischen seinem Gleit- und seinem Steckabschnitt quer zur Führungsbahn erstreckt. Zweckmäßig ist der Steckabschnitt in der zugehörigen Aufnahmeöffnung passgenau form- oder/und kraftschlüssig aufgenommen. Vorzugsweise ist der Gleitabschnitt in der Art einer, insbesondere engen, Spielpassung in dem Kulissenschlitz aufgenommen. Somit lässt sich eine besonders präzise spielarme und trotzdem lösbare relativbewegliche Verbindung zwischen der ersten Befestigungseinrichtung und der Führungseinrichtung erreichen. Entsprechend ist die Übertragung der Ausrichtung eines Resektionsschnittes mittels des chirurgischen Instruments besonders präzise möglich.

In weiterer Ausgestaltung der Erfindung sind zwei Kulissenelemente vorhanden und, insbesondere entlang der Führungsbahn, in einem Abstand zueinander angeordnet. Dies erlaubt eine besonders zuverlässige Führung. Insbesondere wird einem Verkippen der Kulissenelemente in dem Kulissenschlitz entgegengewirkt. Zudem ist die resultierende Steckverbindung vergleichsweise höher belastbar.

In weiterer Ausgestaltung der Erfindung ist ein Trägerelement vorhanden, an welchem das wenigstens eine Kulissenelement befestigt ist. Bevorzugt ist das Kulissenelement stoffschlüssig oder/und reibschlüssig oder/und formschlüssig an dem Trägerelement befestigt. Mittels des Trägerelements kann auf besonders einfache Weise das wenigstens eine Kulissenelement zur Ausbildung der Steckverbindung in die Steckaufnahme und den Kulissenschlitz eingebracht und umgekehrt aus der Steckaufnahme und dem Kulissenschlitz herausgenommen, insbesondere herausgezogen, werden. Mit anderen Worten: Das Trägerelement verbessert die Handhabbarkeit des Kulissenelements zu seiner Montage oder Demontage.

In weiterer Ausgestaltung der Erfindung weist das Trägerelement einen Grundkörper und einen vom Grundkörper, insbesondere orthogonal zur Führungsbahn, abstehenden Griffabschnitt auf. Der Griffabschnitt ist vorzugsweise halbringförmig ausgebildet. Mittels des Griffabschnitts lässt sich das Trägerelement samt dem daran befestigten wenigstens einen Kulissenelement besonders einfach manuell montieren oder demontieren. Ein gesondertes Werkzeug ist hierfür nicht notwendig. Selbst wenn das Kulissenelement oder/und das Trägerelement beispielsweise mit Flüssigkeit benetzt sein sollte, lässt sich der Griffabschnitt sicher und ohne abzurutschen fassen. Ferner lässt sich das Kulissenelement selbst bei einer gegebenenfalls verunreinigungsbedingten Schwergängigkeit komfortabel demontieren.

In weiterer Ausgestaltung der Erfindung umfasst die Kulissenführung, insbesondere der Kulissenschlitz, eine Lochreihe mit mehreren quer zur Führungsbahn axial erstreckten Löchern zum Einrasten des wenigstens einen Kulissenelements, wobei die Löcher entlang der Führungsbahn zueinander beabstandet angeordnet sind und mehrere Raststellungen des wenigstens einen Kulissenelements festlegen. Der zwischen den Löchern vorhandene Abstand bezieht sich dabei vorzugsweise auf Erstreckungsachsen der Löcher. Die Löcher können als Bohrungen ausgebildet sein. Die Mittellängsachsen der Bohrungen können ihren Erstreckungsachsen entsprechen. Die Löcher oder Bohrungen können sich radial berühren oder überschneiden. Insbesondere können sich virtuelle oder tatsächliche Umfangsgeometrien der Löcher oder Bohrungen radial berühren oder überschneiden. Somit lässt sich vorteilhaft eine weitere Funktion in das wenigstens eine Kulissenelement integrieren. Denn zusätzlich zur Führung und zur lösbaren Befestigung dient es bei dieser Ausgestaltung zum Fixieren einer Beweglichkeit der Führungseinrichtung in einer momentanen Raststellung.

In weiterer Ausgestaltung der Erfindung ist der Kulissenschlitz oder/und das wenigstens eine Kulissenelement entgegen einer Rückstellkraft federelastisch verformbar, wobei das wenigstens eine Kulissenelement mittels der Rückstellkraft in seiner momentanen Raststellung gehalten ist. Ein den Kulissenschlitz begrenzendes Material oder/und ein Material des wenigstens einen Kulissenelements kann hierzu federelastisch verformbar ausgebildet sein. Entsprechend kann das Material unter Aufbau der Rückstellkraft einer Belastung in ausreichendem Umfang reversibel nachgeben. Nach Wegnahme der Belastung nimmt es unter Abbau der Rückstellkraft seine ursprüngliche Geometrie ein. Der federelastisch verformbare Kulissenschlitz kann federelastisch aufweitbar sein. Das federelastisch verformbare Kulissenelement kann federelastisch quetschbar sein. Folglich rastet das wenigstens eine Kulissenelement selbsttätig in seiner momentanen Raststellung ein. Entsprechend lässt sich das chirurgische Instrument besonders intuitiv bedienen.

In weiterer Ausgestaltung der Erfindung ist eine Fixiereinrichtung vorhanden, die umschaltbar ist zwischen einem Fixierzustand zur, insbesondere form- oder/und kraftschlüssigen, Fixierung der ersten Befestigungseinrichtung relativ zur Führungseinrichtung und einem Freigabezustand, in welchem die Fixierung aufgehoben, insbesondere gelöst, ist. Vorzugsweise ist in dem Fixierzustand die Beweglichkeit der Führungseinrichtung mittels der Fixiereinrichtung fixiert. Vorteilhaft lässt sich somit eine präzise Anbringung eines mittels des chirurgischen Instruments ausgerichteten Resektionsschnitts sicherstellen. Insbesondere lässt sich durch die Fixiereinrichtung vermeiden, dass durch ein versehentliches Bewegen der Führungseinrichtung relativ zur ersten Befestigungseinrichtung die zuvor korrekte Ausrichtung verfälscht wird.

In weiterer Ausgestaltung der Erfindung weist die Fixiereinrichtung eine parallel zur Führungsbahn erstreckte Zahnreihe und eine Eingriffseinrichtung auf, welche in dem Freigabezustand relativ zur Zahnreihe parallel zur Führungsbahn verstellbeweglich ist, wobei die Zahnreihe an der Führungseinrichtung befestigt und die Eingriffseinrichtung an der ersten Befestigungseinrichtung, insbesondere in wenigstens einer Richtung quer zur Führungsbahn unbeweglich, gelagert ist oder umgekehrt. Eine derartige Fixiereinrichtung erweist sich als besonders robust.

In weiterer Ausgestaltung der Erfindung umfasst die Eingriffseinrichtung ein Formschlusselement, das zum Umschalten zwischen dem Fixier- und dem Freigabezustand zwischen einer Eingriffsstellung und einer Freigabestellung quer zur Führungsbahn verstellbeweglich ist, wobei das Formschlusselement in seiner Eingriffsstellung in die Zahnreihe eingreift und in seiner Freigabestellung ein solcher Eingriff aufgehoben, insbesondere gelöst, ist. Mittels einer solchen Fixiereinrichtung lässt sich die Beweglichkeit der Führungseinrichtung besonders zuverlässig fixieren. Insbesondere ergibt sich ein ausreichend großer Widerstand gegen ein versehentliches Verstellen der Führungseinrichtung.

In weiterer Ausgestaltung der Erfindung ist das Formschlusselement komplementär wenigstens zu einem Teilbereich der Zahnreihe ausgebildet. Dies ermöglicht vorteilhaft eine besonders spielarme Fixierung der Beweglichkeit der Führungseinrichtung.

In weiterer Ausgestaltung der Erfindung umfasst die Eingriffseinrichtung ein federelastisches Vorspannelement zum Erzeugen einer Federkraft, welche Federkraft das Formschlusselement in die Eingriffsstellung zwingt. Das Eingriffselement kann somit selbsttätig seine Eingriffsstellung einnehmen, wenn es nicht absichtlich manuell in seine Freigabestellung gehalten wird. Einem versehentlichen Verstellen des chirurgischen Instruments nach bereits vorgenommener Ausrichtung an der Referenzkomponente wird somit entgegengewirkt.

In weiterer Ausgestaltung der Erfindung umfasst die Eingriffseinrichtung ein blockierbar drehbeweglich gelagertes Zahnrad, welches in die Zahnreihe kämmt und welches in dem Fixierzustand blockiert und in dem Freigabezustand drehbeweglich ist. Damit lässt sich vorteilhaft die Beweglichkeit der Führungseinrichtung wenigstens annäherungsweise stufenlos festsetzen. Das Zahnrad kann reib- oder/und formschlüssig blockierbar sein. Zum reibschlüssigen Blockieren kann beispielsweise eine Axiallagerung des Zahnrads verengt werden, bis das Zahnrad in seiner Axiallagerung reibschlüssig eingeklemmt wird. Zum formschlüssigen Blockieren kann beispielsweise ein gegenüber dem Zahnrad radial verstellbares und drehstarr gelagertes Blockierelement vorhanden sein, welches mit einer Verzahnung des Zahnrads in Eingriff gebracht werden kann.

In weiterer Ausgestaltung der Erfindung weist die Eingriffseinrichtung ein Betätigungselement auf, mittels welchem die Eingriffseinrichtung zum Umschalten zwischen dem Freigabe- und dem Fixierzustand, insbesondere manuell, betätigbar ist. Somit lässt sich das chirurgische Instrument auf besonders intuitive Weise an eine Anatomie des Patienten anpassen. Das Betätigungselement kann einen Mechanismus umfassen, der das Eingriffselement in seiner Eingriffsstellung hält. Ein derartiger Mechanismus kann funktionell und hinsichtlich seines Aufbaus an einen zum Ein- und Ausfahren einer Kugelschreibermine bekannten Mechanismus angelehnt sein. Somit muss der Operateur beim Justieren des chirurgischen Instruments das Betätigungselement nicht dauerhaft gedrückt halten. Ihm stehen also beide Hände zur Verfügung, um die Ausrichtung des anzubringenden Resektionsschnittes vorzunehmen.

In weiterer Ausgestaltung der Erfindung ist das wenigstens eine Kulissenelement durch einen, insbesondere zylindrischen, Stift ausgebildet. Derartige Stifte sind kostengünstig in der Herstellung oder/und als Standardware zukaufbar.

In weiterer Ausgestaltung der Erfindung ist die Führungsbahn innerhalb der sagittalen Führungsebene kreisbogenförmig erstreckt. Vorteilhaft sind die erste Befestigungseinrichtung und die Führungseinrichtung relativ zueinander schwenkbeweglich. In Verwendung des chirurgischen Instruments gemäß dieser Ausgestaltung ist die erste Befestigungseinrichtung lösbar an der femurseitig oder tibiaseitig angebrachten Referenzkomponente befestigt. Der als tibia- oder femurseitige Referenzkomponente fungierende Tibia- oder Femurschnittblock ist lösbar an der zweiten Befestigungseinrichtung befestigt. Die Referenzkomponente ist vorzugsweise ein femorales Probekondylenimplantat. In diesem Fall eignet sich das chirurgische Instrument besonders für eine Verwendung bei der all femur first Variante des KA. Der Femur- oder Tibiaschnittblock kann zur Schnittführung an dem distalen Femur oder an der proximalen Tibia oder dergleichen dienen. Die Führungseinrichtung erlaubt eine geführte Schwenkbewegung der zweiten Befestigungseinrichtung relativ zu der ersten Befestigungseinrichtung. In der Verwendung des chirurgischen Systems erlaubt die Führungseinrichtung folglich eine dementsprechend geführte Relativbewegung des jeweiligen Schnittblocks gegenüber der jeweiligen Referenzkomponente. Hierdurch kann die Neigung des Schnittblocks in der Führungsebene eingestellt werden. Die Führungsebene ist sagittal ausgerichtet und folglich anteroposterior sowie proximodistal erstreckt. Vorzugsweise schneidet eine Schwenkachse, um welche die beiden Befestigungseinrichtungen relativ zueinander mittels der Führungseinrichtung schwenkbeweglich geführt sind, eine mechanische Tibiaachse in der sagittalen Führungsebene. Die besagte Neigung wird auch als posteriorer oder anteriorer Slope bezeichnet. Die Einstellbarkeit der Neigung des Schnittblocks - also des Slopes - erlaubt zum einen eine Anpassung an präoperativ festgelegte Parameter. Zum anderen kann eine tatsächlich vorherrschende Flexions- oder Extensionsstellung des Beins durch die Beweglichkeit der Führungseinrichtung berücksichtigt und ausgeglichen werden. Hierdurch lässt sich sicherstellen, dass eine Einstellung des Slopes nicht gleichzeitig zu einer ungewollten Veränderung einer proximodistalen Position des Schnittblocks und damit einer sogenannten Schnitthöhe führt.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.
- Fig. 1: zeigt eine schematische Perspektivdarstellung einer Ausführungsform eines erfindungsgemäßen chirurgischen Instruments,
- Fig. 2: eine schematische Seitenansicht des chirurgischen Instruments nach Fig. 1,
- Fig. 3: ein Detail der Darstellung nach Fig. 2,
- Fig. 4: ein Detail einer schematischen Perspektivdarstellung des chirurgischen Instruments nach den Fig. 1 bis 3 in einem zur besseren Übersichtlichkeit teilweise zerlegten Zustand,
- Fig. 5: eine schematische Perspektivdarstellung einer ersten Befestigungseinrichtung des chirurgischen Instruments nach den Fig. 1 bis 4,
- Fig. 6: eine schematische Vorderansicht der ersten Befestigungseinrichtung nach Fig. 5,
- Fig. 7: eine schematische Draufsicht der ersten Befestigungseinrichtung nach den Fig. 5 und 6,
- Fig. 8: eine schematische Seitenansicht der ersten Befestigungseinrichtung nach den Fig. 5 bis 7,
- Fig. 9: eine schematische Perspektivdarstellung eines Trägerelements des chirurgischen Instruments nach den Fig. 1 bis 4,
- Fig. 10: eine schematische, gegenüber der Darstellung der Fig. 9 gedrehte Perspektivdarstellung des Griffelements nach Fig. 9,
- Fig. 11: eine schematische Perspektivdarstellung einer weiteren Ausführungsform des erfindungsgemäßen chirurgischen Instruments,
- Fig. 12: eine schematische, gegenüber der Darstellung der Fig. 11 gedrehte schematische Perspektivdarstellung des chirurgischen Instruments nach der Fig. 11,
- Fig. 13: eine schematische Seitenansicht des chirurgischen Instruments nach den Fig. 11 und 12,
- Fig. 14: eine schematische Vorderansicht des chirurgischen Instruments nach den Fig. 11 bis 13,
- Fig. 15: eine schematische Schnittdarstellung bezüglich einer Schnittlinie B-B des chirurgischen Instruments nach den Fig. 11 bis 14,
- Fig. 16: ein Detail der schematischen Seitendarstellung nach Fig. 13,
- Fig. 17: eine schematische Perspektivdarstellung einer Eingriffseinrichtung des chirurgischen Instruments nach den Fig. 11 bis 16,
- Fig. 18: eine schematische Perspektivdarstellung einer ersten Befestigungseinrichtung des chirurgischen Instruments nach den Fig. 11 bis 16,
- Fig. 19: eine schematische, gegenüber der Darstellung der Fig. 18 gedrehte schematische Perspektivdarstellung der ersten Befestigungseinrichtung nach Fig. 18,
- Fig. 20: eine schematische Seitenansicht einer Führungseinrichtung des chirurgischen Instruments nach den Fig. 11 bis 16,
- Fig. 21: eine schematische Untersicht der Führungseinrichtung nach der Fig. 20.

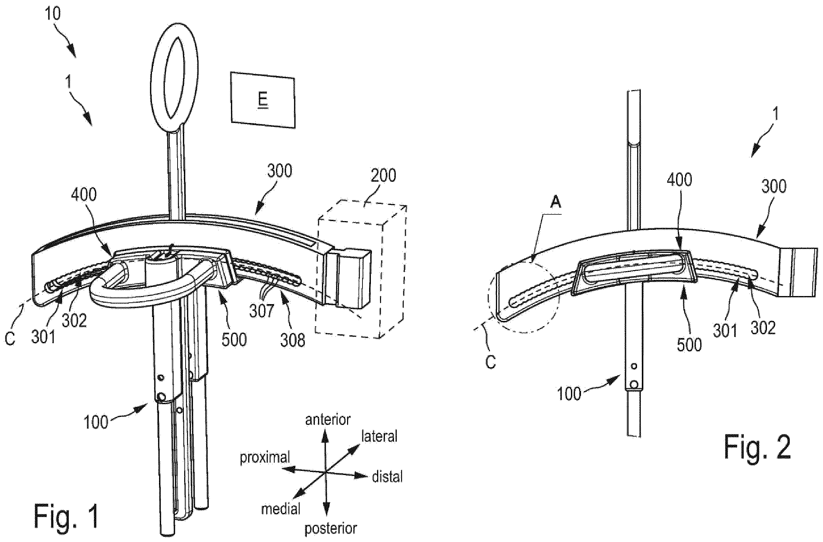

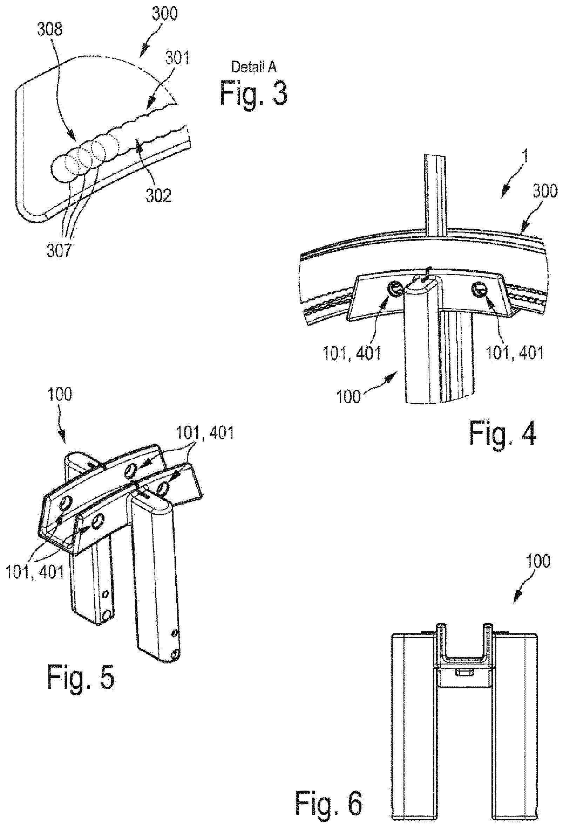

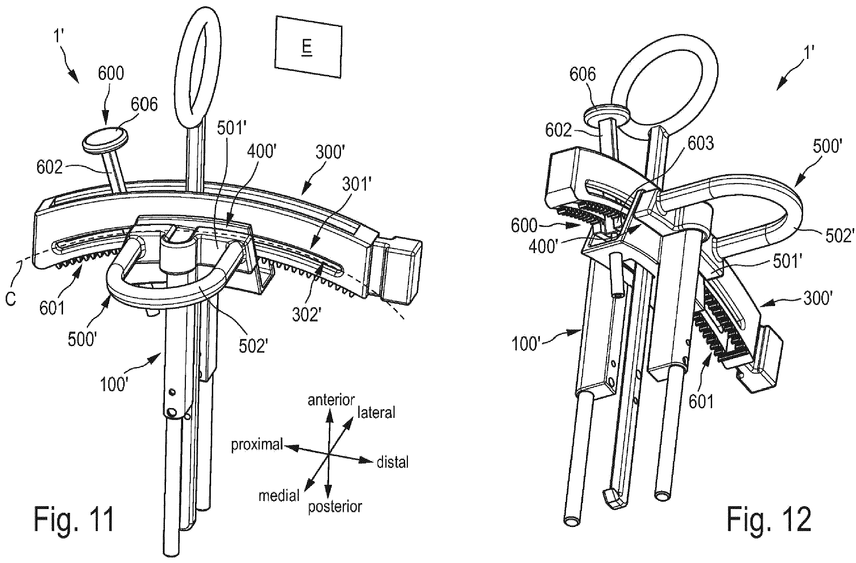

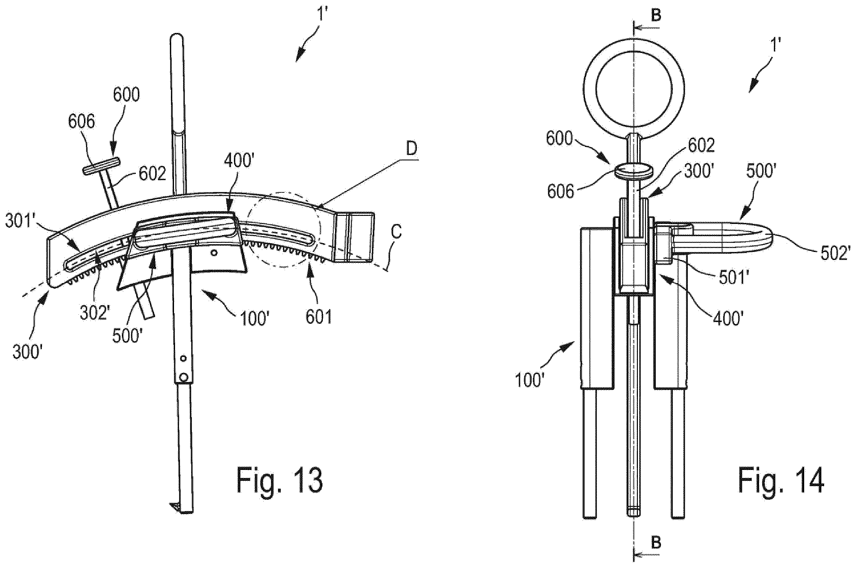

Ein chirurgisches Instrument 1 gemäß Fig. 1 bis 4 ist zur Verwendung bei einer Kniegelenkersatzoperation vorgesehen. Das chirurgische Instrument 1 kann auch als Übertragungswerkzeug oder Tibia Transfer Tool bezeichnet werden. Es dient einer referenzierten Positionierung eines in den Figuren nicht gezeigten Tibiaschnittblocks an einer ebenfalls nicht dargestellten proximalen Tibia. Als Referenz für die Position des Tibiaschnittblocks dient eine bereits an einem distalen Femur positionierte Referenzkomponente - in den Figuren ebenfalls nicht gezeigt. Das chirurgische Instrument 1 eignet sich insbesondere für Kniegelenkersatzoperationen, die dem sogenannten all femur first Ansatz folgen. Gemäß diesem Ansatz werden zunächst sämtliche erforderliche femoralen Resektionsschnitte angebracht. Nach Resektion des distalen Femurs wird an diesem ein femorales Probekondylenimplantat befestigt, welches als femorale Referenzkomponente für das chirurgische Instrument 1 fungiert. Mittels des chirurgischen Instruments 1 lässt sich eine bereits an der Anatomie des Patienten vorgenommene Ausrichtung des Probekondylenimplantats auf eine proximale Tibia transferieren, um dort in korrekter Ausrichtung wenigstens einen Resektionsschnitt vorzunehmen. Alternativ zu dem femoralen Probekondylenimplantat kann als Referenzkomponente auch ein sogenannter Femurschnittblock verwendet werden, falls der Operateur einem anderen als dem all femur first Ansatz folgt. Voranstehendes gilt in gleicher Weise auch für das chirurgische Instrument 1' gemäß den Fig. 11 bis 16.

Die femorale Referenzkomponenten ist nicht Bestandteil des chirurgischen Instruments 1, 1'.

Das chirurgische Instrument 1, 1' umfasst eine erste Befestigungseinrichtung 100, 100'. Die erste Befestigungseinrichtung 100, 100' ist zur lösbaren Befestigung an der femoralen Referenzkomponente eingerichtet. Grundsätzlich kann die erste Befestigungseinrichtung 100, 100' - alternativ oder zusätzlich - auch zur lösbaren Befestigung an einer tibialen Referenzkomponente eingerichtet sein. Das chirurgische Instrument 1, 1' umfasst eine Führungseinrichtung 300, 300'. Die Führungseinrichtung 300, 300' ist einerseits mit der ersten Befestigungseinrichtung 100, 100' lösbar gekoppelt. Andererseits ist die Führungseinrichtung 300, 300' mit einer zweiten Befestigungseinrichtung 200 koppelbar - in der Fig. 1 lediglich exemplarisch angedeutet. Die Führungseinrichtung 300, 300' ermöglicht eine geführte Relativbewegung zwischen der ersten Befestigungseinrichtung 100, 100' und der zweiten Befestigungseinrichtung 200, innerhalb einer sagittalen Führungsebene E. Die zweite Befestigungseinrichtung 200 ist zur lösbaren Befestigung an einer tibialen Komponente eingerichtet. Es versteht sich, dass die zweite Befestigungseinrichtung 200 - alternativ oder zusätzlich - grundsätzlich auch zur lösbaren Befestigung an einer femoralen Komponente eingerichtet sein kann. Mit anderen Worten: Eine tibiale Referenzkomponente wird zusammen mit einer femoralen Komponente und eine femorale Referenzkomponente zusammen mit einer tibialen Komponente verwendet.

Weder die zweite Befestigungseinrichtung 200 noch die tibiale Komponente sind Bestandteil des chirurgischen Instruments 1, 1'.

Die Führungseinrichtung 300, 300' umfasst eine Kulissenführung 301, 301'. Die Kulissenführung 301, 301' weist wenigstens einen entlang einer Führungsbahn C längserstreckten Kulissenschlitz 302, 302' auf. Die Führungsbahn C verläuft in oder parallel zu der Führungsebene E. Die Kulissenführung 301, 301' umfasst ferner wenigstens ein Kulissenelement 303, 303', 303a, 303a', 303b, 303b', welches entlang der Führungsbahn C gleitbeweglich in dem Kulissenschlitz 302, 302' aufgenommen ist. Die erste Befestigungseinrichtung 100, 100' weist wenigstens eine orthogonal zur Führungsbahn C erstreckte Steckaufnahme 101, 101' auf. Durch diese wenigstens eine Steckaufnahme 101, 101' ist das wenigstens eine Kulissenelement 303, 303', 303a, 303a', 303b, 303b' unter Ausbildung einer lösbaren Steckverbindung 400, 400' mit der ersten Befestigungseinrichtung 100, 100' in den wenigstens einen Kulissenschlitz 302, 302' eingebracht. Die Steckverbindung 400, 400' dient zur Kopplung der ersten Befestigungseinrichtung 100, 100' mit der Führungseinrichtung 300, 300'. Dem Kulissenelement 303, 303', 303a, 303a', 303b, 303b' kommt somit eine Doppelfunktion zu: einerseits die Ausbildung der lösbaren Steckverbindung 400, 400', andererseits die Führung im Kulissenschlitz 302, 302'. Vorliegend weist das chirurgische Instrument 1, 1' zwei Kulissenelemente 303, 303', 303a, 303a', 303b, 303b' auf. Die beiden Kulissenelemente 303, 303', 303a, 303a', 303b, 303b' sind im Abstand zueinander angeordnet, beispielsweise entlang der Führungsbahn C. Jedem Kulissenelement 303, 303', 303a, 303a', 303b, 303b' kann eine Steckaufnahme 101, 101' zugeordnet sein.

Vorliegend ist die Führungsbahn C innerhalb der sagittalen Führungsebene E kreisbogenförmig erstreckt. Somit sind die erste Befestigungseinrichtung 100, 100' und die Führungseinrichtung 300, 300' relativ zueinander innerhalb der Führungsebene E schwenkbeweglich. Wenn an der Führungseinrichtung 300, 300' eine zweite Befestigungseinrichtung 200 befestigt ist, sind somit die erste und die zweite Befestigungseinrichtung 100, 100', 200 mittels der Führungseinrichtung 300, 300' innerhalb der Führungsebene E relativ zueinander schwenkbeweglich geführt.

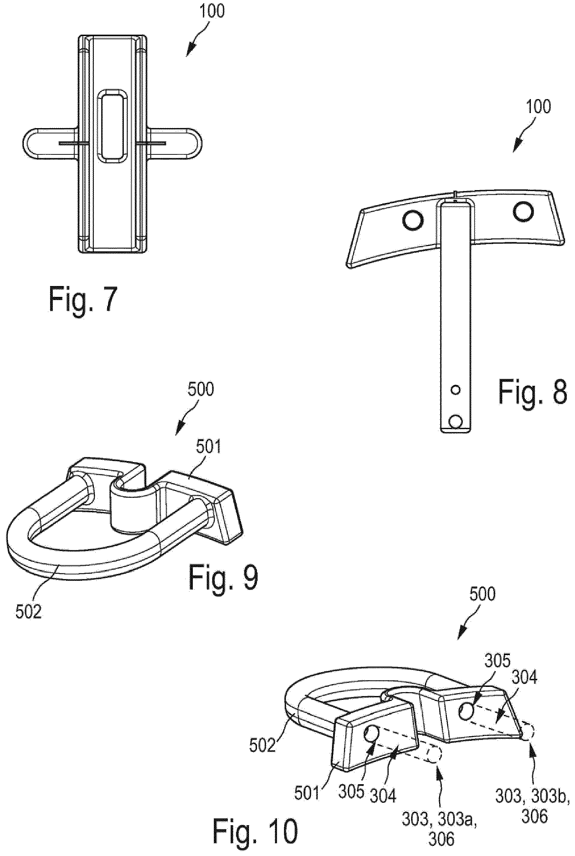

Fig. 10 zeigt, dass die Kulissenelemente 303, 303a, 303b jeweils einen Gleit- und einen Steckabschnitt 304, 305 aufweisen. Dabei sind die Kulissenelemente 303, 303a, 303b vorliegend zwischen dem Gleit- und dem Steckabschnitt 304, 305 quer zur Führungsbahn C erstreckt. Entsprechendes gilt für die Kulissenelemente 303', 303a', 303b'. Der Gleitabschnitt 304 greift zur gleitbeweglichen Führung entlang der Führungsbahn C in den Kulissenschlitz 302, 302' ein. Der Steckabschnitt 305 ist in einer zugehörigen Aufnahmeöffnung 401, 401' der Steckaufnahme 400, 400' lösbar aufgenommen. Der Steckabschnitt 305 kann passgenau in der zugehörigen Aufnahmeöffnung 401, 401' lösbar aufgenommen sein. Der Steckabschnitt 305 kann unter Ausbildung einer Übermaßpassung in der zugehörigen Aufnahmeöffnung 401, 401' aufgenommen sein.

Das chirurgische Instrument 1, 1' umfasst ein Trägerelement 500, 500', gesondert gezeigt in den Fig. 9 und 10. Das wenigstens eine Kulissenelement 303, 303', 303a, 303a', 303b, 303b' ist an dem Trägerelement 500, 500' befestigt. Vorliegend sind beide Kulissenelemente 303, 303', 303a, 303a', 303b, 303b' an dem Trägerelement 500, 500' befestigt. Die Befestigung des wenigstens einen Kulissenelements 303, 303', 303a, 303a', 303b, 303b' kann stoffschlüssig oder/und reibschlüssig oder/und formschlüssig umgesetzt sein. Beispielsweise kann das wenigstens eine Kulissenelement 303, 303', 303a, 303a', 303b, 303b' in einer zugehörigen Bohrung des Trägerelements 500, 500' eingepresst sein. Alternativ können das Trägerelement 500, 500' und das wenigstens eine Kulissenelement 303, 303', 303a, 303a', 303b, 303b' integral aneinander ausgebildet sein. Das Trägerelement 500, 500' erlaubt es vorliegend, beide Kulissenelemente 303, 303', 303a, 303a', 303b, 303b' gleichzeitig aus dem Kulissenschlitz 302, 302' und den zugehörigen Steckaufnahmen 101, 101' zu entfernen. So lässt sich die Steckverbindung 400, 400' mit nur einem Handgriff zu lösen. Es versteht sich, dass das Trägerelement 500, 500' in umgekehrter Weise auch eine gleichzeitige Montage beider Kulissenelemente 303, 303', 303a, 303a', 303b, 303b' zur Ausbildung der Steckverbindung 400, 400' erlaubt. Das Trägerelement 500, 500' umfasst einen Grundkörper 501, 501'. Das Trägerelement 500, 500' umfasst ferner einen Griffabschnitt 502, 502', welcher von dem Grundkörper 501, 501' absteht. Vorliegend steht der Griffabschnitt 502, 502' orthogonal zur Führungsbahn C von dem Grundkörper 501, 501' ab. Der Griffabschnitt 502, 502' ist vorliegend halbringförmig ausgebildet. In dem halbringförmigen Griffabschnitt 502, 502' ist wenigstens ein menschlicher Finger zum abrutschsicheren Greifen des Griffabschnitts 502, 502' aufnehmbar. Das Trägerelement 500, 500' lässt sich vorliegend medial mitsamt den daran befestigten Kulissenelementen 303, 303', 303a, 303a', 303b, 303b' demontieren. Es versteht sich, dass die Montage zur Ausbildung der Steckverbindung 400, 400' in umgekehrter Richtung, also lateral erfolgt.

Bei der Ausführungsform des chirurgischen Instruments 1 gemäß den Fig. 1 bis 4 weist die Kulissenführung 301 eine Lochreihe 308 auf. Dabei ist die Lochreihe 308 vorliegend an dem Kulissenschlitz 302 vorhanden. Die Lochreihe 308 umfasst mehrere quer zur Führungsbahn C erstreckte Löcher 307. Die Löcher 307 dienen zum Einrasten des wenigstens einen Kulissenelements 303, 303a, 303b. Vorliegend können beide Kulissenelemente 303, 303a, 303b in den Löchern 307 einrasten. Die Löcher 307 sind vorliegend mediolateral längserstreckt. Die Löcher 307 sind entlang der Führungsbahn C zueinander im Abstand angeordnet. Vorliegend sind die Löcher 307 äquidistant zueinander angeordnet. Die Löcher 307 legen mehrere Raststellungen der Kulissenelemente 303, 303a, 303b fest. Der Abstand zwischen den Löchern 307 bezieht sich dabei auf ihre Erstreckungsachsen. Somit können die Löcher 307 wie vorliegend als einander radial überschneidende Bohrungen ausgebildet sein. Es versteht sich, dass die Löcher 307 alternativ als einander radial oder tangential berührende Bohrungen oder als einander nicht berührende Bohrungen ausgebildet sein können. Der Kulissenschlitz 302 sowie - alternativ oder zusätzlich - das wenigstens eine Kulissenelement 303, 303a, 303b ist vorliegend entgegen einer Rückstellkraft federelastisch verformbar. Dabei ist das wenigstens eine Kulissenelement 300, 303a, 303b mittels der Rückstellkraft in seiner momentanen Raststellung gehalten. Somit muss zum Verstellen des Kulissenelements 303, 303a, 303b von seiner momentanen Raststellung in eine entlang der Führungsbahn C nächstgelegene Raststellung die Rückstellkraft zwischenzeitlich überwunden werden.

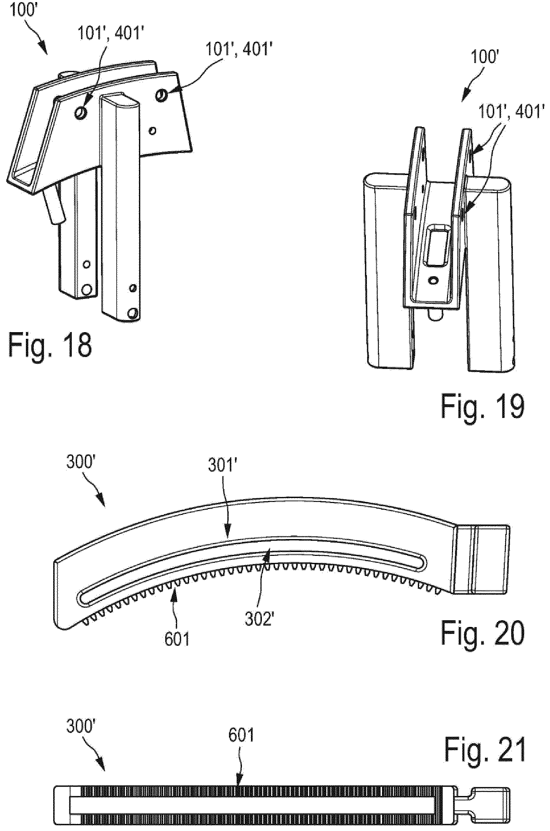

Bei der Ausführungsform gemäß den Fig. 11 bis 16 weist das chirurgische Instrument 1' eine Fixiereinrichtung 600 auf. Die Fixiereinrichtung 600 ist umschaltbar zwischen einem Fixierzustand und einem Freigabezustand. Der Fixierzustand dient zur Fixierung der ersten Befestigungseinrichtung 100' relativ zur Führungseinrichtung 300'. In dem Freigabezustand ist diese Fixierung aufgehoben oder gelöst. Es kann sich um eine form- oder/und kraftschlüssige Fixierung handeln. In dem Fixierzustand ist die Beweglichkeit der Führungseinrichtung 300' mittels der Fixiereinrichtung 600 fixiert. Die Fixiereinrichtung 600 umfasst eine Zahnreihe 601 sowie eine Eingriffseinrichtung 602. Dabei ist die Zahnreihe 601 parallel zur Führungsbahn C erstreckt. Zähne und Zahnzwischenräume der Zahnreihe 601 sind im Wechsel parallel zur Führungsbahn aufgereiht. Die Eingriffseinrichtung 602 ist in dem Freigabezustand relativ zur Zahnreihe 601 parallel zur Führungsbahn C verstellbeweglich. Die Zahnreihe 601 ist an der Führungseinrichtung 300' befestigt. Die Eingriffseinrichtung 602 ist an der ersten Befestigungseinrichtung 100' gelagert. Dabei ist die Eingriffseinrichtung 602 quer zur Führungsbahn C in wenigstens einer Richtung - vorliegend sowohl mediolateral als auch proximodistal - unbeweglich gelagert. Die Eingriffseinrichtung 602 ist vorliegend anteroposterior linear beweglich gelagert. Es versteht sich, dass in umgekehrter Weise alternativ die Eingriffseinrichtung 602 an der Führungseinrichtung 303 gelagert und die Zahnreihe 601 an der ersten Befestigungseinrichtung 100' befestigt sein kann, was in den Figuren aber nicht gezeigt ist. Die Eingriffseinrichtung 602 umfasst ein Formschlusselement 603. Das Formschlusselement 603 ist zum Umschalten zwischen dem Fixier- und dem Freigabezustand zwischen einer Eingriffsstellung und einer Freigabestellung quer zur Führungsbahn C verstellbeweglich. In der Eingriffsstellung greift das Formschlusselement 603 in die Zahnreihe 601 ein. Demgegenüber ist ein solcher Eingriff in der Freigabestellung aufgehoben oder gelöst. Das Formschlusselement 603 kann zu wenigstens einem Teilbereich der Zahnreihe 601 komplementär ausgebildet sein. Vorliegend weist das Formschlusselement 602 einen zu einem Teilbereich der Zahnreihe 601 komplementäre Zahnung auf. Gemäß den Fig. 11 bis 21 sind zwei parallel zueinander angeordnete Zahnreihen 601 mit je einer zugehörigen Eingriffseinrichtung 602 mit Formschlusselement 603 vorhanden.

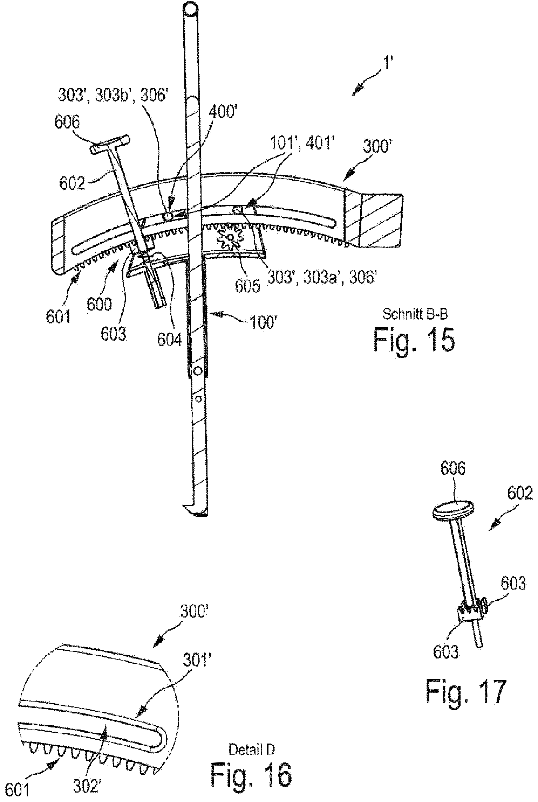

Die Eingriffseinrichtung 602 umfasst ein federelastisches Vorspannelement 604 zum Erzeugen einer Federkraft - siehe Fig. 15. Diese Federkraft zwingt das Formschlusselement 603 in seine Eingriffsstellung. Somit muss die Federkraft überwunden werden, um das Formschlusselement 603 in die Freigabestellung zu bewegen. Wie vorliegend kann die Eingriffseinrichtung 602 ein blockierbar drehbeweglich gelagertes Zahnrad 605 umfassen. Das Zahnrad 605 kämmt in die Zahnreihe 601. In dem Fixierzustand ist das Zahnrad 605 blockiert. Demgegenüber ist das Zahnrad 605 in dem Freigabezustand relativ zur Zahnreihe 601 drehbeweglich. Alternativ oder zusätzlich zum blockierbaren Zahnrads 605 kann ein in den Figuren nicht gezeigtes unblockierbares Zahnrad vorhanden sein, welches zur Unterstützung der Relativführung von Führungseinrichtung 300' und erster Befestigungseinrichtung 100' dient. Vorliegend umfasst die Eingriffseinrichtung 602 ferner ein Betätigungselement 606. Mittels des Betätigungselements 606 ist die Eingriffseinrichtung 602 zum Umschalten zwischen dem Freigabe- und dem Fixierzustand - beispielsweise manuell - betätigbar. Das Betätigungselement 606 ist vorliegend in der Art eines drückbaren Knopfs ausgebildet. Es ist dabei denkbar, das Betätigungselement 606 mit einem "Kugelschreibermechanismus" auszustatten. Ein solcher Mechanismus verhindert ein selbsttätiges Rückstellen des Formschlusselements 603 in seine Eingriffsstellung. Er muss zum Verstellen in die Freigabestellung ein erstes Mal und zum Rückstellen in die Eingriffsstellung ein zweites Mal betätigt werden und umgekehrt.

Das wenigstens eine Kulissenelement 303, 303', 303a, 303a', 303b, 303b' ist vorliegend durch einen Stift 306, 306' ausgebildet. Vorliegend sind beide Kulissenelemente 303, 303', 303a, 303a', 303b, 303b' jeweils durch einen Stift 306, 306' gebildet. Beispielsweise handelt es sich um einen zylindrischen Stift 306, 306'. Der Stift 306, 306' kann an wenigstens einem seiner axialen Enden geschlitzt oder/und ungeschlitzt sein. Wenn wie vorliegend zwei stiftartige Kulissenelemente 303, 303', 303a, 303a', 303b, 303b' vorhanden sind, kann einer der Stifte 306, 306' geschlitzt und einer ungeschlitzt sein kann. Ist der Stift 306, 306' geschlitzt, so kann ein Außendurchmesser des geschlitzten Stifts 306, 306' an dem geschlitzten axialen Ende erweitert sein gegenüber einem restlichen Bereich des Stifts 306, 306'. Somit lässt sich eine Übermaßpassung bereitstellen, die den geschlitzten Stift 306, 306' in seiner montierten Position an der Führungseinrichtung 300, 300' hält, und zwar ohne, dass eine Gängigkeit der Führungseinrichtung 300, 300' beeinträchtigt wird.

Das chirurgische Instrument 1 und die zweite Befestigungseinrichtung 200 sind Bestandteile eines chirurgischen Instrumentensystems 10. Das chirurgische Instrumentensystem 10 kann darüber hinaus die femorale Referenzkomponente oder/und die tibiale Komponente umfassen.

## Patentansprüche

1. Chirurgisches Instrument (1, 1') zur Verwendung bei einer Kniegelenkersatzoperation, aufweisend
eine erste Befestigungseinrichtung (100, 100'), welche zur lösbaren Befestigung an einer femoralen Referenzkomponente eingerichtet ist,
eine Führungseinrichtung (300, 300'), welche zum einen mit der ersten Befestigungseinrichtung (100, 100') lösbar gekoppelt und zum anderen mit einer zweiten Befestigungseinrichtung (200), welche zur lösbaren Befestigung an einer tibialen Komponente eingerichtet ist, koppelbar ist und eine in einer sagittalen Führungsebene (E) geführte Relativbewegung zwischen der ersten Befestigungseinrichtung (100, 100') und der zweiten Befestigungseinrichtung (200) ermöglicht,
wobei die Führungseinrichtung (300, 300') eine Kulissenführung (301, 301') mit wenigstens einem entlang einer Führungsbahn (C) längserstreckten Kulissenschlitz (302, 302') und wenigstens einem Kulissenelement (303, 303', 303a, 303a', 303b, 303b') aufweist, welches entlang der Führungsbahn (C) gleitbeweglich in dem Kulissenschlitz (302, 302') aufgenommen ist,
und wobei die erste Befestigungseinrichtung (100, 100') wenigstens eine orthogonal zu der Führungsbahn (C) erstreckte Steckaufnahme (101, 101') aufweist, durch welche das wenigstens eine Kulissenelement (303, 303', 303a, 303a', 303b, 303b') unter Ausbildung einer lösbaren Steckverbindung (400, 400') mit der ersten Befestigungseinrichtung (100, 100') in den wenigstens einen Kulissenschlitz (302, 302') eingebracht ist.

2. Chirurgisches Instrument (1, 1') nach Anspruch 1, **dadurch gekennzeichnet, dass** das wenigstens eine Kulissenelement (303, 303', 303a, 303a', 303b, 303b') einen Gleit- und einem Steckabschnitt (304, 305) aufweist, wobei zur gleitbeweglichen Führung entlang der Führungsbahn (C) der Gleitabschnitt (304) in den Kulissenschlitz (302, 302') eingreift und der Steckabschnitt (305) in einer zugehörigen Aufnahmeöffnung (401, 401') der Steckaufnahme (101, 101') lösbar aufgenommen ist.

3. Chirurgisches Instrument (1, 1') nach Anspruch 1 oder 2, **dadurch gekennzeichnet, dass** zwei Kulissenelemente (303, 303', 303a, 303a', 303b, 303b') vorhanden und, insbesondere entlang der Führungsbahn (C), in einem Abstand zueinander angeordnet sind.

4. Chirurgisches Instrument (1, 1') nach einem der vorhergehenden Ansprüche, **dadurch gekennzeichnet, dass** ein Trägerelement (500, 500') vorhanden ist, an welchem das wenigstens eine Kulissenelement (303, 303', 303a, 303a', 303b, 303b') befestigt ist.

5. Chirurgisches Instrument (1, 1') nach Anspruch 4, **dadurch gekennzeichnet, dass** das Trägerelement (500, 500') einen Grundkörper (501, 501') und einen vom Grundkörper (501, 501'), insbesondere orthogonal zur Führungsbahn (C), abstehenden Griffabschnitt (502, 502') aufweist.

6. Chirurgisches Instrument (1) nach einem der vorhergehenden Ansprüche, **dadurch gekennzeichnet, dass** die Kulissenführung (301), insbesondere der Kulissenschlitz (302), eine Lochreihe (308) mit mehreren quer zur Führungsbahn (C) axial erstreckten Löchern (307) zum Einrasten des wenigsten einen Kulissenelements (303, 303a, 303b) umfasst, wobei die Löcher (307) entlang der Führungsbahn (C) zueinander beabstandet angeordnet sind und mehrere Raststellungen des wenigstens einen Kulissenelements (303, 303a, 303b) festlegen.

7. Chirurgisches Instrument (1) nach Anspruch 6, **dadurch gekennzeichnet, dass** der Kulissenschlitz (302) oder/und das wenigstens eine Kulissenelement (303, 303a, 303b) entgegen einer Rückstellkraft federelastisch verformbar ist, wobei das wenigstens eine Kulissenelement (303, 303a, 303b) mittels der Rückstellkraft in seiner momentanen Raststellung gehalten ist.

8. Chirurgisches Instrument (1') nach einem der vorhergehenden Ansprüche, **dadurch gekennzeichnet, dass** eine Fixiereinrichtung (600) vorhanden ist, die umschaltbar ist zwischen einem Fixierzustand zur Fixierung der Relativbeweglichkeit zwischen der ersten Befestigungseinrichtung (100') und der Führungseinrichtung (300') und einem Freigabezustand, in welchem die Fixierung aufgehoben ist.

9. Chirurgisches Instrument (1') nach Anspruch 8, **dadurch gekennzeichnet, dass** die Fixiereinrichtung (600) eine parallel zur Führungsbahn (C) erstreckte Zahnreihe (601) und eine Eingriffseinrichtung (602) aufweist, welche in dem Freigabezustand relativ zur Zahnreihe (601) parallel zur Führungsbahn (C) verstellbeweglich ist, wobei die Zahnreihe (601) an der Führungsreinrichtung (300') befestigt und die Eingriffseinrichtung (602) an der ersten Befestigungseinrichtung (100'), insbesondere in wenigstens einer Richtung quer zur Führungsbahn (C) unbeweglich, gelagert ist oder umgekehrt.

10. Chirurgisches Instrument (1') nach Anspruch 9, **dadurch gekennzeichnet, dass** die Eingriffseinrichtung (602) ein Formschlusselement (603) umfasst, das zum Umschalten zwischen dem Fixier- und dem Freigabezustand zwischen einer Eingriffsstellung und einer Freigabestellung quer zur Führungsbahn (C) verstellbeweglich ist, wobei das Formschlusselement (603) in seiner Eingriffsstellung in die Zahnreihe (601) eingreift und in seiner Freigabestellung ein solcher Eingriff aufgehoben ist.

11. Chirurgisches Instrument (1') nach Anspruch 10, **dadurch gekennzeichnet, dass** die Eingriffseinrichtung (602) ein federelastisches Vorspannelement (604) zum Erzeugen einer Federkraft umfasst, welche Federkraft das Formschlusselement (603) in die Eingriffsstellung zwingt.

12. Chirurgisches Instrument (1') nach einem der Ansprüche 9 bis 11, **dadurch gekennzeichnet, dass** die Eingriffseinrichtung (602) ein blockierbar drehbeweglich gelagertes Zahnrad (605) umfasst, welches in die Zahnreihe (601) kämmt und welches in dem Fixierzustand blockiert und in dem Freigabezustand drehbeweglich ist.

13. Chirurgisches Instrument (1') nach einem der Ansprüche 9 bis 12, **dadurch gekennzeichnet, dass** die Eingriffseinrichtung (602) ein Betätigungselement (606) aufweist, mittels welchem die Eingriffseinrichtung (602) zum Umschalten zwischen dem Freigabe- und dem Fixierzustand, insbesondere manuell, betätigbar ist.

14. Chirurgisches Instrument (1, 1') nach einem der vorhergehenden Ansprüche, **dadurch gekennzeichnet, dass** das wenigstens eine Kulissenelement (303, 303', 303a, 303a', 303b, 303b') durch einen, insbesondere zylindrischen, Stift (306, 306') ausgebildet ist.

15. Chirurgisches Instrument (1, 1') nach einem der vorhergehenden Ansprüche, **dadurch gekennzeichnet, dass** die Führungsbahn (C) innerhalb der sagittalen Führungsebene (E) kreisbogenförmig erstreckt ist.

## Claims

1. Surgical instrument (1, 1') for use in a total knee arthroplasty, comprising
a first fastening device (100, 100') which is configured for releasable fastening to a femoral reference component,
a guide device (300, 300') which is firstly releasably coupled to the first fastening device (100, 100') and secondly can be coupled to a second fastening device (200) which is configured for releasable fastening to a tibial component, and allows a relative movement guided in a sagittal guide plane (E) between the first fastening device (100, 100') and the second fastening device (300, 300'),
wherein the guide device (300, 300') comprises a sliding guide (301, 301') with at least one guide slot (302, 302') longitudinally extending along a guide track (C), and at least one sliding member (303, 303', 303a, 303a, 303b, 303b') which is received in the guide slot (302, 302') so as to be slidingly movable along the guide track (C),
and wherein the first fastening device (100, 100') has at least one push-fit receiver (101, 101') which extends orthogonally to the guide track (C) and via which the at least one sliding member (303, 303', 303a, 303a, 303b, 303b') is introduced into the at least one guide slot (302, 302'), forming a releasable push-fit connection (400, 400') with the first fastening device (100, 100').

2. Surgical instrument (1, 1') according to Claim 1, **characterized in that** the at least one sliding member (303, 303', 303a, 303a, 303b, 303b') has a sliding portion (304) and a push-fit portion (305), wherein for slidingly movable guidance along the guide track (C), the sliding portion (304) engages in the guide slot (302, 302') and the push-fit portion (305) is releasably received in an associated receiving opening (401, 401') of the push-fit receiver (101, 101').

3. Surgical instrument (1, 1') according to Claim 1 or 2, **characterized in that** two sliding members (303, 303', 303a, 303a, 303b, 303b') are present and spaced apart from one another, in particular along the guide track (C).

4. Surgical instrument (1, 1') according to any of the preceding claims, **characterized in that** a carrier element (500, 500') is present on which the at least one sliding member (303, 303', 303a, 303a, 303b, 303b') is fastened.

5. Surgical instrument (1, 1') according to Claim 4, **characterized in that** the carrier element (500, 500') has a base body (501, 501') and a handle portion (502, 502') protruding from the base body (501, 501'), in particular orthogonally to the guide track (C).

6. Surgical instrument (1) according to any of the preceding claims, **characterized in that** the sliding guide (301), in particular the guide slot (302), comprises a hole row (308) with several holes (307) extending axially transversely to the guide track (C) for latching of the at least one sliding member (303, 303a, 303b), wherein the holes (C) are spaced apart from one another along the guide track (C) and establish several latching positions for the at least one sliding member (303, 303a, 303b).

7. Surgical instrument (1) according to Claim 6, **characterized in that** the guide slot (302) and/or the at least one sliding member (303, 303a, 303b) is spring-elastically deformable against a return force, wherein the at least one sliding member (303, 303a, 303b) is held in its momentary latching position by means of the return force.

8. Surgical instrument (1') according to any of the preceding claims, **characterized in that** a fixing device (600) is provided which is switchable between a fixing state for fixing the relative movability between the first fastening device (100') and the guide device (300'), and a release state in which the fixing is cancelled.

9. Surgical instrument (1') according to Claim 8, **characterized in that** the fixing device (300) comprises a tooth row (601) extending parallel to the guide track (C), and an engagement device (602) which, in the release state, is adjustably movable relative to the tooth row (601) parallel to the guide track (C), wherein the tooth row (601) is fastened to the guide device (300') and the engagement device (602) is mounted on the first fastening device (100'), in particular immovably in at least one direction transversely to the guide track (C), or vice versa.

10. Surgical instrument (1') according to Claim 9, **characterized in that** the engagement device (602) comprises a form-fit element (603) which is adjustably movable transversely to the guide track (C) between an engagement position and a release position for switching between the fixing state and the release state, wherein the form-fit element (603) in its engagement position engages in the tooth row (601) and in its release position, such an engagement is cancelled.

11. Surgical instrument (1') according to Claim 10, **characterized in that** the engagement device (602) comprises a biasing member (604) which biases the form-fit element (603) into the engagement position.

12. Surgical instrument (1') according to one of Claims 9 to 11, **characterized in that** the engagement device (602) comprises a gearwheel (605) which is mounted so as to be blockable and rotationally movable, and which meshes in the tooth row (601) and blocks in the fixing state and is rotationally movable in the release state.

13. Surgical instrument (1') according to any of Claims 9 to 12, **characterized in that** the engagement device (602) comprises an actuating element (606) by means of which the engagement device (602) can be actuated, in particular manually, for switching between the release state and the fixing state.

14. Surgical instrument (1, 1') according to any of the preceding claims, **characterized in that** the at least one sliding member (303, 303', 303a, 303a, 303b, 303b') is a, in particular cylindrical, pin (306, 306').

15. Surgical instrument (1, 1') according to any of the preceding claims, **characterized in that** the guide track (C) extends in arcuate form within the sagittal guide plane (E).

## Revendications

1. Instrument chirurgical (1, 1') destiné à être utilisé lors d'une opération de remplacement de l'articulation du genou, comprenant :
un premier dispositif de fixation (100, 100') conçu pour être fixé de manière amovible à un composant de référence fémoral,
un dispositif de guidage (300, 300') qui, d'une part, est couplé de manière amovible au premier dispositif de fixation (100, 100') et, d'autre part, peut être couplé à un second dispositif de fixation (200), conçu pour être fixé de manière amovible à un composant tibial, et qui permet un mouvement relatif entre le premier dispositif de fixation (100, 100') et le second dispositif de fixation (200), guidé dans un plan de guidage sagittal (E),
le dispositif de guidage (300, 300') présentant un guidage à coulisse (301, 301') comportant au moins une fente de coulisse (302, 302') s'étendant longitudinalement le long d'une trajectoire de guidage (C) et au moins un élément de coulisse (303, 303', 303a, 303a', 303b, 303b') reçu de manière coulissante dans la fente de coulisse (302, 302') le long de la trajectoire de guidage (C),
et le premier dispositif de fixation (100, 100') présentant au moins un logement d'emboîtement (101, 101') s'étendant orthogonalement à la trajectoire de guidage (C), à travers lequel l'au moins un élément de coulisse (303, 303', 303a, 303a', 303b, 303b') est introduit dans l'au moins une fente de coulisse (302, 302') en formant une liaison par emboîtement amovible (400, 400') avec le premier dispositif de fixation (100, 100').

2. Instrument chirurgical (1, 1') selon la revendication 1, **caractérisé en ce que** l'au moins un élément de coulisse (303, 303', 303a, 303a', 303b, 303b') présente une section coulissante (304) et une section d'emboîtement (305), la section coulissante (304) venant en prise dans la fente de coulisse (302, 302') pour assurer un guidage coulissant le long de la trajectoire de guidage (C), et la section d'emboîtement (305) étant reçue de manière amovible dans une ouverture de réception associée (401, 401') du logement d'emboîtement (101, 101').

3. Instrument chirurgical (1, 1') selon la revendication 1 ou 2, **caractérisé en ce que** deux éléments de coulisse (303, 303', 303a, 303a', 303b, 303b') sont présents et sont disposés à distance l'un de l'autre, notamment le long de la trajectoire de guidage (C).

4. Instrument chirurgical (1, 1') selon l'une des revendications précédentes, **caractérisé en ce qu'**un élément de support (500, 500') est présent, sur lequel l'au moins un élément de coulisse (303, 303', 303a, 303a', 303b, 303b') est fixé.

5. Instrument chirurgical (1, 1') selon la revendication 4, **caractérisé en ce que** l'élément de support (500, 500') présente un corps de base (501, 501') et une section de préhension (502, 502') faisant saillie à partir du corps de base (501, 501'), notamment orthogonalement à la trajectoire de guidage (C).

6. Instrument chirurgical (1) selon l'une des revendications précédentes, **caractérisé en ce que** le guidage à coulisse (301), notamment la fente de coulisse (302), comprend une rangée de trous (308) comportant plusieurs trous (307) s'étendant axialement transversalement à la trajectoire de guidage (C) et destinés à l'encliquetage de l'au moins un élément de coulisse (303, 303a, 303b), les trous (307) étant disposés à distance les uns des autres le long de la trajectoire de guidage (C) et définissant plusieurs positions d'encliquetage de l'au moins un élément de coulisse (303, 303a, 303b).

7. Instrument chirurgical (1) selon la revendication 6, **caractérisé en ce que** la fente de coulisse (302) et/ou l'au moins un élément de coulisse (303, 303a, 303b) sont déformables de manière élastique à l'encontre d'une force de rappel, l'au moins un élément de coulisse (303, 303a, 303b) étant maintenu dans sa position d'encliquetage actuelle au moyen de la force de rappel.

8. Instrument chirurgical (1') selon l'une des revendications précédentes, **caractérisé en ce qu'**un dispositif de blocage (600) est présent, lequel peut être commuté entre un état de blocage destiné à bloquer le mouvement relatif entre le premier dispositif de fixation (100') et le dispositif de guidage (300') et un état de libération dans lequel le blocage est supprimé.

9. Instrument chirurgical (1') selon la revendication 8, **caractérisé en ce que** le dispositif de blocage (600) présente une rangée de dents (601) s'étendant parallèlement à la trajectoire de guidage (C) et un dispositif de mise en prise (602) qui, dans l'état de libération, est mobile par rapport à la rangée de dents (601), parallèlement à la trajectoire de guidage (C), la rangée de dents (601) étant fixée au dispositif de guidage (300') et le dispositif de mise en prise (602) étant monté sur le premier dispositif de fixation (100'), notamment de manière immobile dans au moins une direction transversale à la trajectoire de guidage (C), ou inversement.

10. Instrument chirurgical (1') selon la revendication 9, **caractérisé en ce que** le dispositif de mise en prise (602) comprend un élément d'accouplement par complémentarité de formes (603), qui est mobile transversalement à la trajectoire de guidage (C), entre une position de mise en prise et une position de libération, afin de permettre la commutation entre l'état de blocage et l'état de libération, l'élément d'accouplement par complémentarité de formes (603) venant en prise dans la rangée de dents (601) dans sa position de mise en prise, une telle mise en prise étant supprimée dans sa position de libération.

11. Instrument chirurgical (1') selon la revendication 10, **caractérisé en ce que** le dispositif de mise en prise (602) comprend un élément de précontrainte élastique (604) destiné à générer une force élastique qui sollicite l'élément d'accouplement par complémentarité de formes (603) vers la position de mise en prise.

12. Instrument chirurgical (1') selon l'une des revendications 9 à 11, **caractérisé en ce que** le dispositif de mise en prise (602) comprend une roue dentée (605) montée de manière rotative et pouvant être bloquée, laquelle engrène avec la rangée de dents (601) et est bloquée dans l'état de blocage et mobile en rotation dans l'état de libération.

13. Instrument chirurgical (1') selon l'une des revendications 9 à 12, **caractérisé en ce que** le dispositif de mise en prise (602) présente un élément d'actionnement (606) au moyen duquel le dispositif de mise en prise (602) peut être actionné, notamment manuellement, afin de permettre la commutation entre l'état de libération et l'état de blocage.

14. Instrument chirurgical (1, 1') selon l'une des revendications précédentes, **caractérisé en ce que** l'au moins un élément de coulisse (303, 303', 303a, 303a', 303b, 303b') est constitué par une broche (306, 306'), notamment cylindrique.

15. Instrument chirurgical (1, 1') selon l'une des revendications précédentes, **caractérisé en ce que** la trajectoire de guidage (C) s'étend en arc de cercle dans le plan de guidage sagittal (E).
